# NEW EUROPEAN PATENT SPECIFICATION

(11) **EP 0 619 726 B2**
(45) Date of publication and mention of the opposition decision: **21.06.2000**
(45) Mention of the grant of the patent: 16.04.1997
(21) Application number: 93901426.2
(22) Date of filing: 29.12.1992
(51) Int. Cl.: A61F 13/15, A61F 13/54

(54) **AN ABSORBENT ARTICLE**
ABSORBIERENDER ARTIKEL
PRODUIT ABSORBANT

(30) Priority: 30.12.1991 SE 9103853
(43) Date of publication of application: 19.10.1994
(73) Proprietor: SCA Mölnlycke AB, 405 03 Göteborg (SE)
(72) Inventor: BÖHM, Thomas, S-421 69 Västra Frölunda (SE)
(74) Representative: Romare, Laila Anette
(86) International application number: SE9200898
(87) International publication number: WO9312745

(56) References cited:
- EP-A- 0 268 858
- EP-A- 0 523 683
- EP-A- 0 543 116
- EP-AB- 0 523 719
- GB-A- 2 244 653
- JP-A- 11 021 191
- JP-A- 25 171 892
- SE-B- 412 508
- SE-B- 432 348
- US-A- 4 780 352

## Description

The present invention relates to an absorbent product, such as a sanitary napkin, a diaper or an incontinence guard or the like, comprising an absorbent body enclosed between a liquid-impermeable bottom layer and at least one liquid-permeable top layer, consisting of a net, a perforated film or a non-woven material, which during use of the product faces the user's body.

Liquid-permeable top layers consisting of plastic nets or perforated plastic film are previously known. The rewetting properties of such top layers are good, and the surface is thus felt to be dry and comfortable. One disadvantage is, however, that the edge of the net or the plastic film can rub sharply against the skin.

The purpose of the present invention is, in a simple and inexpensive manner, to solve the above problem and provide a product which utilizes the good re-wetting properties of these materials and at the same time provides increased smoothness and comfort. This is achieved by virtue of the fact that the liquid-permeable top layer terminates somewhat inside the longitudinal lateral edges of the product. Such a top sheet terminating somewhat inside the longitudinal lateral edges of the product is already known from SE-B-412508. According to the invention the longitudinal lateral edges of the top layer are covered by and fixed to folds of a soft skin-friendly material, e.g. a non-woven material which is fixed to the bottom layer. The soft skin-friendly material also extends across the absorbent body between this body and the top layer, thus forming together with the top layer a double upper covering layer for the product. The soft skin-friendly material can consist of a less exclusive and thus cheaper material than the material in the top layer.

By virtue of the fact that the edges of the top layer are covered, these edges will not rub against the user's skin. According to the invention, the longitudinal lateral edges of the top layer are covered by folds of a soft non-woven material, which is fixed to the bottom layer. The edge of these folds forms the liquid barrier against lateral leakage. These barriers can, according to an additional variant of the invention, be made higher with the aid of elastic threads or tapes laid in the folds.

The soft skin-friendly material also extends across the absorbent body inside the top layer and has portions which extend outside the absorbent body and are joined to the corresponding portions of the bottom layer extending beyond the absorbent body. This provides a double surface-material, a laminate. The inventive fixing of the top layer along the lateral edges makes it possible to eliminate or almost eliminate the need for adhesion to the inner top layer, and this makes the double surface material according to the invention smoother than a conventionally laminated double surface material. The secure anchoring provided by the lateral fixing of the top layer also avoids the risk of "delamination", i.e. that the top layer and the non-woven material inside it will be separated from each other.

The invention will be described below in more detail with reference to several examples shown in the accompanying drawings, where:
Fig. 1 shows schematically an embodiment of a sanitary napkin according to the invention in a view from above,
Fig. 2 shows a section along the line II-II in Fig. 1,
Fig. 3 shows a cross-section of a variant of the sanitary napkin in Fig. 1.

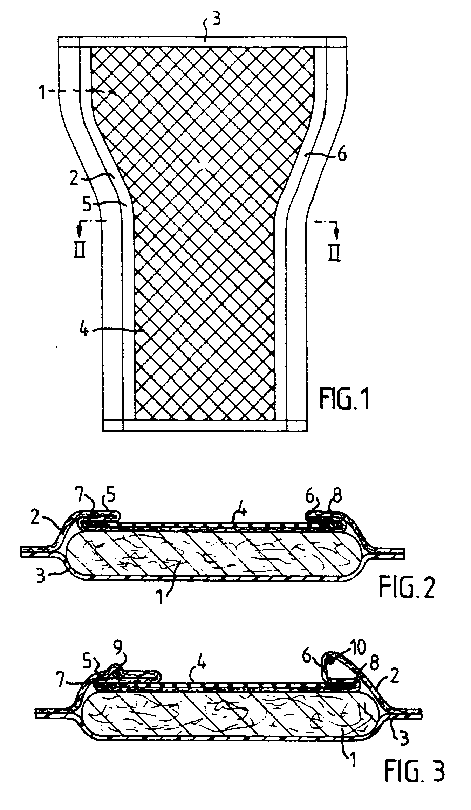

Figs. 1 and 2 show a sanitary napkin according to one embodiment of the invention. This sanitary napkin comprises in a conventional manner an absorbent body 1 enclosed between a liquid-permeable layer 2, which suitably consists of a soft non-woven material which faces the user's body during use, and a liquid-impermeable bottom layer 3. The layers 2 and 3 have portions which extend beyond the absorbent body and the layers are joined to each other in these portions. The bottom layer 3 consists of a suitable plastic material, i.e. polyethylene. The absorbent body is preferably made of fluff-pulp with or without so-called superabsorbents mixed in.

Other known materials for the absorbent body and the top and bottom layers can of course be used within the scope of the invention.

According to the examples shown in the drawing, a liquid-permeable top layer 4 in the form of a net or the like is placed outside the non-woven layer 2 above the portion thereof which covers the top side of the absorbent body 1, i.e. the side of the absorbent body which faces the user during use of the sanitary napkin. The net 4 is made of a non-absorbent material. The material is preferably hydrophilic, at least on the side facing the user during use in order to allow the liquid to easily flow through the material. The opposite side can possibly be hydrophobic. If the opening area of the net is sufficiently large, even completely hydrophobic materials can be used. Suitable materials for the net are synthetic materials, e.g. polypropylene or polyethylene. Instead of a net, a perforated, flat or three-dimensional plastic film of for example polypropylene or polyethylene can be used. The holes should preferably have a diameter of 0.1 to 3 mm. The outer top layer 4 can also be of a non-woven material based on polymer fibers, e.g. polypropylene or polyethylene, dtex 1-10. By virtue of the fact that the outer top layer 4 terminates somewhat inside the lateral edges of the napkin, a saving in materials is obtained.

The net 4 is fixed to the non-woven layer 2 along its longitudinal edges by folds 5, 6 in the inner top layer. These folds are folded over the longitudinal lateral edges of the net 4 and are then fixed to the same and to the portion of the inner top layer 2 in contact with the top of the absorbent body 1. This fixing can be achieved with the aid of strings of adhesive 7, 8, as indicated in Fig. 2, but heat or ultrasonic welding can also be used. A very strong attachment of the net 4 is thereby obtained at the same time as the risk is eliminated of the net edges rubbing against the user's skin.

In the embodiment shown in Figs. 1 and 2, the portions of the bottom layer 3 and the non-woven layer 2 extending beyond the ends of the absorbent body 1, and which are joined to each other, are folded over the absorbent body 1 and the end edges of the net 4 and are fixed to the net and the underlying portions of the non-woven layer 2. In order to avoid the liquid-impermeable bottom layer 3 from extending beyond the side of the sanitary napkin which faces the user, the bottom layer 3 can be dimensioned so that it is shorter than the inner layer 2 and when folded in only covers the end edges of the absorbent body 1, while the longer non-woven layer 2 extends so that it also covers the end edges of the net 4. It is also possible to make the non-woven layer 2 with transverse folds analogous to the longitudinal folds 5, 6. It is also possible to use separate tapes of skin-friendly material to cover the end edges of the net 4.

By virtue of the fact that the net 4 is securely anchored to the non-woven layer 2 along the lateral edges, only a relatively weak bond is required between the net and the non-woven layer as compared to a simple laminated material. The smoothness of the surface layer is in general dependent on how hard the two layers 2 and 4 are fixed to each other, and therefore a weaker bond provides a softer surface.

Even if it is preferable to join the net 4 to the underlying inner top layer 2 in the manner described above, it is not absolutely necessary since the edge of the folds 5, 6 act as a barrier against lateral leakage. In the embodiment according to Fig. 3, this effect has been amplified by elastic threads 9, 10 being laid into the folds in the stretched state and fixed to the inner top layer 2 at least at the ends thereof. In the left hand portion of Fig. 3, there is shown the configuration of the fold 5 during the manufacture of the sanitary napkin, i.e. when it is held flat and streched out, while the right hand portion of Fig. 3 shows the configuration of the fold 6 after the elastic thread 10 has been allowed to contract and thus deform the sanitary napkin to its curved form. By making the folds larger than in the embodiment shown in Figs. 1 and 2, relatively high liquid barriers are created. The size of the folds 5, 6 thus determines the height of the liquid barriers.

The folds 5, 6 can also be arranged outside the absorbent body 1, which can be suitable if they are provided with elastic threads 9, 10. In this case, the net 4 will extend slightly outside the absorbent body.

Having, as shown in Figs. 2 and 3, double cover layers 2 and 4, the distance is increased between the absorbent body 1 and the user's skin. The non-woven layer 2 acts as a spatial layer and can possibly be porous, which reduces rewetting. Only a small amount of liquid will stick in the net openings, and this is particularly important in sanitary napkins due to the high viscosity of menstrual fluid. The surface is experienced both visually and by touch as being dry.

The invention can of course be applied to the type of sanitary napkin where the non-woven material extends around the entire napkin, even outside the liquid-impermeable bottom layer 3. In such a case, the sanitary napkin lacks the lateral edges of the cover layers 2, 3 extending outside the absorbent body.

Even if the invention is particularly suited to sanitary napkins where the viscosity of the menstrual fluid and the proximity to the lateral edges of the sanitary napkin from the wetting point accentuates the risk of lateral leakage, the invention can also be utilized with advantage in other types of absorbent disposable articles, such as incontinence guards and diapers.

## Claims

1. Absorbent product such as a sanitary napkin, a diaper or an incontinence guard or the like, comprising an absorbent body (1) enclosed between a liquid-impermeable bottom layer (3) and at least one liquid-permeable top layer (4), consisting of a net, a perforated film or a non-woven material, which during use of the product faces the user body and which terminates somewhat inside the longitudinal lateral edges of the product, **characterized** in that the longitudinal lateral edges of the top layer (4) are covered by and fixed to folds (5,6) of a soft, skin-friendly material (2), e.g. a non-woven material which is fixed to the bottom layer (3), and in that the soft skin-friendly material (2) also extends across the absorbent body (1) between this body and the top layer (4), thus forming together with the top layer a double upper covering layer for the product.

2. Product according to Claim 1, **characterized** in that the top layer (4) is joined to the soft skin-friendly material (2) not only at the lateral edges but otherwise as well.

3. Product according to one of Claims 1 or 2, **characterized** in that elastic threads (9,10) in a stretched state are laid in the folds (5,6) and are fixed to the soft, skin-friendly material (2), said elastic threads (9,10) in contracted state curve the absorbent body (1) longitudinally and lift the central portions of the folds (5,6) so that they form longitudinal liquid barriers.

## Patentansprüche

1. Absorptionsartikel, insbesondere Hygienebinde, Windel, Inkontinenzschutz oder ähnliches, mit einem Absorptionskörper (1), der zwischen einer flüssigkeitsdurchlässigen Unterschicht (3) und wenigstens einer flüssigkeitsundurchlässigen Oberschicht (4) eingeschlossen ist, die aus einem Netz, einer löcherigen Folie oder einem Vliesstoff besteht, die während der Verwendung des Artikels zu dem Körper des Benutzers gerichtet ist und ein wenig innerhalb der Längsseitenränder des Artikels endet, dadurch **gekennzeichnet**, daß die Längsseitenränder der Oberschicht (4) mit Falzen (5, 6) eines weichen, hautfreundlichen Materials (2), insbesondere eines Vliesstoffs, das an der Unterschicht (3) befestigt ist, bedeckt sind und an diesen befestigt sind und daß das weiche, hautfreundliche Material (2) sich ferner über den Absorptionskörper (1) zwischen diesem Körper und der Oberschicht (4) erstreckt, wodurch zusammen mit der Oberschicht eine doppelte obere Abdeckschicht für den Artikel ausgebildet wird.

2. Artikel nach Anspruch 1, dadurch **gekennzeichnet**, daß die Oberschicht (4) mit dem weichen, hautfreundlichen Material (2) nicht nur an den Seitenrändern, sondern auch anderweitig verbunden ist.

3. Artikel nach Anspruch 1 oder 2, dadurch **gekennzeichnet**, daß elastische Fäden (9, 10) in einem gedehnten Zustand in den Falzen (5, 6) verlegt sind, und an dem weichen, hautfreundlichen Material (2) befestigt sind, wobei die elastischen Fäden (9, 10) in einem zusammengezogenen Zustand den Absorptionskörper (1) in Längsrichtung krümmen und die mittleren Abschnitte der Falze (5, 6) anheben, so daß sie längsverlaufende Flüssigkeitsbarrieren bilden.

## Revendications

1. Article absorbant comme une serviette hygiénique, une couche-culotte, une protection pour incontinents ou analogue, comprenant un corps absorbant (1) enfermé entre une couche inférieure (3) imperméable aux liquides et au moins une couche supérieure (4) perméable aux liquides, comprenant un filet, un film perforé ou un matériau non tissé, qui se trouve contre le corps de l'utilisateur quand l'article est porté et qui se termine quelque peu a l'intérieur des bords latéraux longitudinaux de l'article, caractérisé en ce que les bords latéraux longitudinaux de la couche supérieure (4) sont recouverts et fixés par des plis (5, 6) d'un matériau (2) doux et agréable pour la peau comme un matériau non-tissé qui est fixé à la couche inférieure (3) et en ce que le matériau (2) doux agréable pour la peau s'étend aussi sur le corps absorbant (1), entre ce corps et la couche supérieure (4), formant ainsi avec la couche supérieure une double couche de couverture supérieure pour l'article.

2. Article selon la revendication 1, caractérisé en ce que la couche supérieure (4) est réunie au matériau (2) doux et agréable pour la peau non seulement sur les bords latéraux mais en d'autres endroits aussi.

3. Article selon la revendication 1 ou 2, caractérisé en ce que des fils élastiques (9, 10) en état de pré-extension sont disposés dans les plis (5, 6) et sont fixés au matériau (2) doux et agréable pour la peau, lesdits fils élastiques (9, 10) à l'état contracté faisant courber le corps absorbant (1) dans le sens longitudinal et soulever les parties centrales des plis (5, 6) de sorte qu'ils forment des barrières longitudinales pour le liquide.
